# EUROPEAN PATENT APPLICATION

(11) **EP 1 852 054 A1**
(43) Date of publication of application: **07.11.2007**
(21) Application number: 07251813.7
(22) Date of filing: 30.04.2007
(51) Int. Cl.: A61B 1/005

(54) **Medical instrument having an end-effector-associated member**

(30) Priority: 01.05.2006 US 415451
(71) Applicant: ETHICON ENDO-SURGERY, INC., Cincinnati, Ohio 45242 (US)
(72) Inventor: Lu, Ifung, Skokie, IL 60076 (US); Nobis, Rudolph H., Mason, OH 45040 (US); Vakharia, Omar, Cincinnati, OH 45040 (US)
(74) Representative: Tunstall, Christopher Stephen

(57) **Abstract**

A medical instrument includes a medical-end-effector-associated member and resiliently flexible first, second and third elongate members. The medical-end-effector-associated member has a rigid portion positionable within a patient. The first, second and third elongate members each have a proximal end portion and a distal end portion. The distal end portions of the first, second and third elongate members are attached to the rigid portion of the medical-end-effector-associated member. Relative lengthwise translation of the proximal end portions of the first, second and third elongate members articulates the rigid portion of the medical-end-effector-associated member.

## Description

**Field of the Invention**

The present invention is related generally to medical equipment, and more particularly to a medical instrument having a medical-end-effector-associated member.

**Background of the Invention**

Endoscopes (including colonoscopes) are known which have an insertion tube which is insertable within a patient. The insertion tube has an articulatable distal end portion controlled by wires running from the distal end portion to control knobs on the handle of the endoscope. A wide angle video camera in the distal end of the insertion tube permits medical observation. A medical needle-knife assembly is part of a known endoscopic system and includes a medical end effector in the form of a medical needle-knife attached to an activation wire with both needle-knife and wire surrounded by a flexible shaft. The shaft is insertable into a working channel of the insertion tube of the endoscope and is translatable to the distal end portion of the endoscope insertion tube. Then, the wire is lengthwise translated to extend the medical needle-knife from the shaft and from the distal end portion of the endoscope insertion tube. Then, in one example, the medical needle-knife is used to provide medical treatment by energizing the wire with energy from a radio-frequency generator. A medical grasper assembly is known which includes a medical end effector in the form of a medical grasper having a proximal portion attached to a flexible shaft and includes an activation wire disposed in a lumen of the shaft and operatively connected to a pivotal mechanism of the end effector, wherein lengthwise translation of the activation wire opens and closes the medical grasper.

Still, scientists and engineers continue to seek improved medical instruments having a medical-end-effector-associated member.

**Summary of the Invention**

A first expression of an embodiment of the invention is for a medical instrument including a medical-end-effector-associated member and resiliently flexible first, second and third elongate members. The medical-end-effector-associated member has a rigid portion positionable within a patient. The first, second and third elongate members each have a proximal end portion and a distal end portion. The distal end portions of the first, second and third elongate members are attached to the rigid portion of the medical-end-effector-associated member. Relative lengthwise translation of the proximal end portions of the first, second and third elongate members articulates the rigid portion of the medical-end-effector-associated member.

A second expression of an embodiment of the invention is for a medical instrument including a medical-end-effector-associated member and resiliently flexible first, second and third coilpipes. The medical-end-effector-associated member has a rigid portion positionable within a patient. The first, second and third coilpipes each have a proximal end portion and a distal end portion. The distal end portions of the first, second and third coilpipes are attached to the rigid portion of the medical-end-effector-associated member. Relative lengthwise translation of the proximal end portions of the first, second and third coilpipes articulates the rigid portion of the medical-end-effector-associated member.

A third expression of an embodiment of the invention is for a medical instrument including a medical-end-effector-associated member, resiliently flexible first, second and third coilpipes, and a medical-end-effector activation cable. The medical-end-effector-associated member has a rigid portion positionable within a patient. The first, second and third coilpipes each have a proximal end portion and a distal end portion. The distal end portions of the first, second and third coilpipes are attached to the rigid portion of the medical-end-effector-associated member. Relative lengthwise translation of the proximal end portions of the first, second and third coilpipes articulates the rigid portion of the medical-end-effector-associated member. The activation cable is positionable in one of the first, second and third coilpipes.

Several benefits and advantages are obtained from one or more of the expressions of an embodiment of the invention. In a first application, the medical-end-effector-associated member is inserted into a working channel of a flexible insertion tube of an endoscope, wherein the medical-end-effector-associated member can be articulated with respect to the insertion tube of the endoscope allowing independent alignment of the wide angle video camera of the endoscope and the medical-end-effector-associated member. In a second application, the medical-end-effector-associated member is adapted to be coupled to, and slid along, an exterior rail of a flexible insertion tube of an endoscope allowing independent alignment of the wide angle video camera of the endoscope and the medical-end-effector-associated member. In one example, the medical-end-effector-associated member is a medical end effector such as, without limitation, a medical grasper. In another example, the medical-end-effector-associated member has a passageway, and the medical instrument also includes a medical end effector, such as, without limitation, a medical needle-knife which is translatable through, distally extendable from, and proximally retractable into the passageway.

The following is a non-exhaustive list of embodiments of the invention that are or may be claimed in this application or in subsequently filed divisional applications.

Embodiment 1. A medical instrument comprising: a) a medical-end-effector-associated member having a rigid portion disposable within a patient; and b) resiliently flexible first, second and third elongate members each having a proximal end portion and a distal end portion, wherein the distal end portions of the first, second and third elongate members are attached to the rigid portion of the medical-end-effector-associated member, and wherein relative lengthwise translation of the proximal end portions of the first, second and third elongate members articulates the rigid portion of the medical-end-effector-associated member.

Embodiment 2. The medical instrument of embodiment 1, wherein the rigid portion of the medical-end-effector-associated member has a proximal side, and wherein the distal end portions of the first, second and third elongate members have centerlines which intersect the proximal side at points which define vertices of a substantially equilateral triangle.

Embodiment 3. The medical instrument of embodiment 1, wherein the medical-end-effector-associated member and the distal end portions of the first, second and third elongate members are disposable within a working channel of an endoscope insertion tube.

Embodiment 4. The medical instrument of embodiment 1, wherein the first, second and third elongate members consist essentially of nitinol.

Embodiment 5. The medical instrument of embodiment 1, wherein the medical-end-effector-associated member is a medical end effector.

Embodiment 6. The medical instrument of embodiment 5, also including a medical end effector attached to the rigid portion of the medical-end-effector-associated member.

Embodiment 7. The medical instrument of embodiment 1, wherein the rigid portion of the medical-end-effector-associated member has a proximal side, a distal side, and a passageway extending from the proximal side to the distal side, and also including a medical end effector translatable through, distally extendable from, and proximally retractable into the passageway.

Embodiment 8. A medical instrument comprising: a) a medical-end-effector-associated member having a rigid portion disposable within a patient; and b) resiliently flexible first, second and third coilpipes each having a proximal end portion and a distal end portion, wherein the distal end portions of the first, second and third coilpipes are attached to the rigid portion of the medical-end-effector-associated member, and wherein relative lengthwise translation of the proximal end portions of the first, second and third coilpipes articulates the rigid portion of the medical-end-effector-associated member.

Embodiment 9. The medical instrument of embodiment 8, wherein the rigid portion of the medical-end-effector-associated member has a proximal side, and wherein the distal end portions of the first, second and third coilpipes have coil centerlines which intersect the proximal side at points which define vertices of a substantially equilateral triangle.

Embodiment 10. The medical instrument of embodiment 8, wherein the medical-end-effector-associated member and the distal end portions of the first, second and third coilpipes are disposable within a working channel of an endoscope insertion tube.

Embodiment 11. The medical instrument of embodiment 8, wherein the first, second and third coilpipes consist essentially of nitinol.

Embodiment 12. The medical instrument of embodiment 8, wherein the medical-end-effector-associated member is a medical end effector.

Embodiment 13. The medical instrument of embodiment 8, wherein the rigid portion of the medical-end-effector-associated member has a proximal side, a distal side, and a passageway extending from the proximal side to the distal side, and also including a medical end effector translatable through, distally extendable from, and proximally retractable into the passageway.

Embodiment 14. A medical instrument comprising: a) a medical-end-effector-associated member having a rigid portion disposable within a patient; b) resiliently flexible first, second and third coilpipes each having a proximal end portion and a distal end portion, wherein the distal end portions of the first, second and third coilpipes are attached to the rigid portion of the medical-end-effector-associated member, and wherein relative lengthwise translation of the proximal end portions of the first, second and third coilpipes articulates the rigid portion of the medical-end-effector-associated member; and c) a medical-end-effector activation cable disposable in one of the first, second and third coilpipes.

Embodiment 15. The medical instrument of embodiment 14, wherein the rigid portion of the medical-end-effector-associated member has a proximal side, and wherein the distal end portions of the first, second and third coilpipes have coil centerlines which intersect the proximal side at points which define vertices of a substantially equilateral triangle.

Embodiment 16. The medical instrument of embodiment 14, wherein the medical-end-effector-associated member and the distal end portions of the first, second and third coilpipes are disposable within a working channel of an endoscope insertion tube.

Embodiment 17. The medical instrument of embodiment 14, wherein the first, second and third coilpipes consist essentially of nitinol.

Embodiment 18. The medical instrument of embodiment 14, wherein the medical-end-effector-associated member is a medical end effector, and wherein the activation cable is disposed in one of the first, second and third coilpipes and is operatively connected to the medical end effector.

Embodiment 19. The medical instrument of embodiment 18, also including a medical end effector attached to the rigid portion of the medical-end-effector-associated member, wherein the activation cable is disposed in one of the first, second and third coilpipes and is operatively connected to the medical end effector.

Embodiment 20. The medical instrument of embodiment 14, wherein the rigid portion of the medical-end-effector-associated member has a proximal side, a distal side, and a passageway extending from the proximal side to the distal side, and also including a medical end effector attached to the activation cable, wherein distal lengthwise translation of the activation cable distally extends the medical end effector from the passageway and proximal lengthwise translation of the activation cable proximally retracts the medical end effector into the passageway.

The present invention has, without limitation, application in hand-activated instruments as well as in robotic-assisted instruments.

**Brief Description of the Figures**

FIGURE 1 is a schematic, side elevational, cross sectional view of an embodiment of a medical instrument of the invention wherein the medical-end-effector-associated member is a medical end effector and wherein the medical end effector includes a medical grasper;

FIGURE 2 is a cross sectional view of the medical instrument of Figure 1 taken along lines 2-2 in Figure 1 with the activation cable omitted for clarity;

FIGURE 3 is a view, as in Figure 1, but also showing the medical-end-effector-associated member disposed in a working channel of an endoscope insertion tube;

FIGURE 4 is a view, as in Figure 1, but of a first alternate embodiment of a medical instrument wherein the medical instrument includes a medical end effector attached to the medical-end-effector-associated member;

FIGURE 5 is a cross sectional view of the medical instrument of Figure 4 taken along lines 5-5 in Figure 4 with the activation cable omitted for clarity;

FIGURE 6 is a view, as in Figure 1, but of a second alternate embodiment of a medical instrument wherein the medical instrument includes a medical end effector, wherein the medical end effector includes a medical needle-knife which is translatable through and distally extendable from a passageway of the medical-end-effector-associated member; and

FIGURE 7 is a cross sectional view of the medical instrument of Figure 6 taken along lines 7-7 in Figure 6 with the activation cable omitted for clarity.

**Detailed Description of the Invention**

Before explaining the present invention in detail, it should be noted that the invention is not limited in its application or use to the details of construction and arrangement of parts illustrated in the accompanying drawings and description. The illustrative embodiments of the invention may be implemented or incorporated in other embodiments, variations and modifications, and may be practiced or carried out in various ways. Furthermore, unless otherwise indicated, the terms and expressions employed herein have been chosen for the purpose of describing the illustrative embodiments of the present invention for the convenience of the reader and are not for the purpose of limiting the invention.

It is understood that any one or more of the following-described embodiments, examples, etc. can be combined with any one or more of the other following-described embodiments, examples, etc.

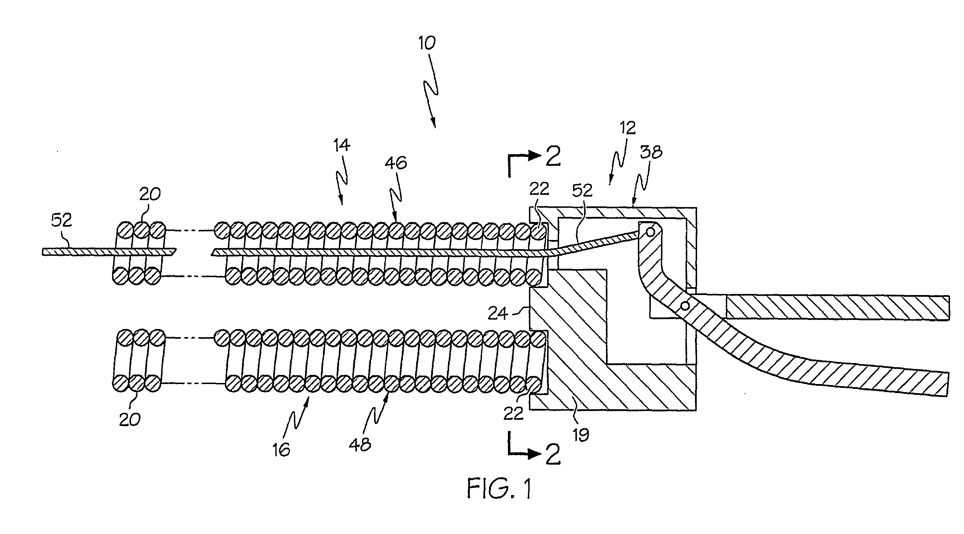

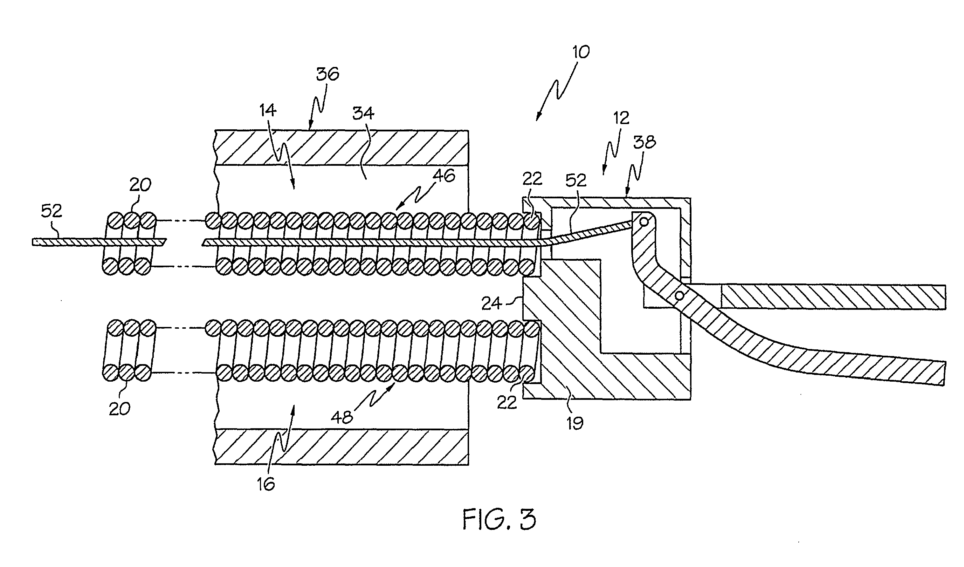

Referring now to the Figures, wherein like numerals represent like elements throughout, Figures 1-3 illustrate an embodiment of the invention. A first expression of the embodiment of Figures 1-3 is for a medical instrument 10 including a medical-end-effector-associated member 12 and resiliently flexible first, second and third elongate members 14, 16 and 18. The medical-end-effector-associated member 12 has a rigid portion 19 disposable within a patient. The first, second and third elongate members 14, 16 and 18 each have a proximal end portion 20 and a distal end portion 22. The distal end portions 22 of the first, second and third elongate members 14, 16 and 18 are attached to the rigid portion 19 of the medical-end-effector-associated member 12. Relative lengthwise translation of the proximal end portions 20 of the first, second and third elongate members 14, 16 and 18 articulates the rigid portion 19 of the medical-end-effector-associated member 12.

It is noted that a rigid portion 19 is a portion of the medical-end-effector-associated member 12 which cannot be manually bent by an average-strength adult person. It is also noted that in a first example the entire medical-end-effector-associated member is a rigid portion and that in a second example one or more portions of the medical-end-effector-associated member are rigid portions and one or more other portions of the medical-end-effector-associated member are not rigid portions. Likewise, a flexible elongate member is an elongate member which can be manually bent by an average-strength adult person.

A medical-end-effector-associated member 12 is a member which is a medical end effector (such as, without limitation, a medical grasper type of medical end effector), or is a member (such as, without limitation, a platform base) which is attached to a medical end effector (such as, without limitation, a medical grasper type of medical end effector), or is a member (such as, without limitation, a platform guide) which has a passageway wherein a medical end effector (such as, without limitation, a medical needle-knife type of medical end effector) is translatable through and is distally extendable from the passageway. It is noted that substantially equal lengthwise translation of the proximal end portions 20 of the first, second and third elongate members 14, 16 and 18 will translate the medical-end-effector-associated member 12 without articulation. It also is noted that relative lengthwise translation of the proximal end portion 20 of one of the first, second and third elongate members 14, 16 and 18 with respect to the other two elongate members or relative lengthwise translation of the proximal end portion 20 of two of the first, second and third elongate members 14, 16 and 18 with respect to the other one elongate member will articulate the medical-end-effector-associated member 12.

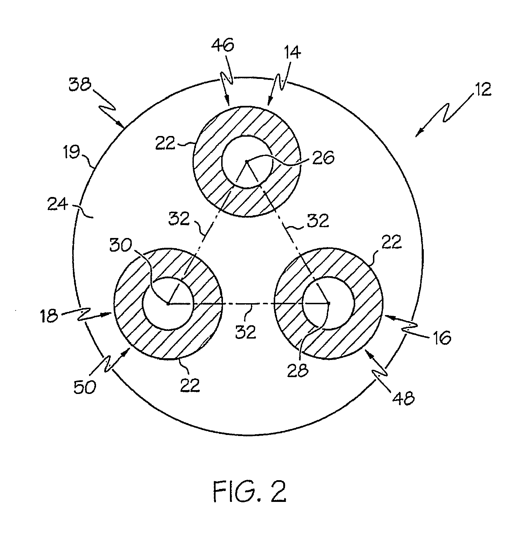

In one enablement of the first expression of the embodiment of Figures 1-3, the rigid portion 19 of the medical-end-effector-associated member 12 has a proximal side 24, wherein the distal end portions 22 of the first, second and third elongate members 14, 16 and 18 have centerlines which intersect the proximal side 24 at points 26, 28 and 30 which define vertices of a substantially equilateral triangle. It is noted that the centerlines are seen on end as the points 26, 28 and 30 in Figure 2 and that straight lines (shown as dashed lines) 32 drawn connecting the points 26, 28 and 30 define the sides of the substantially equilateral triangle.

In one implementation of the first expression of the embodiment of Figures 1-3, the medical-end-effector-associated member 12 and the distal end portions 22 of the first, second and third elongate members 14, 16 and 18 are disposable within a working channel 34 of an endoscope insertion tube 36. In one employment, the working channel 34 has a diameter of substantially 3.2 millimeters.

In one choice of materials, the first, second and third elongate members 14, 16 and 18 consist essentially of nitinol. It is noted that nitinol is a superelastic having shape memory properties wherein the nitinol can have a desired shape set into the first, second and third elongate members 14, 16 and 18 and wherein after flexing the elongate members, the elongate members will resiliently return to their set shape, as is known to those skilled in the art. In one variation, not shown, the first, second and third elongate members each are wires which are substantially straight in a relaxed state. The term "wire" includes a monolithic wire, a braided wire, and a wire having wire segments lengthwise attached end to end. It is noted that a monolithic wire consists of one continuous wire piece.

In one application of the first expression of the embodiment of Figures 1-3, the medical-end-effector-associated member 12 is a medical end effector 38. In one variation, the distal end portions 22 of the first, second and third elongate members 14, 16 and 18 are attached to the rigid portion 19 of the medical end effector 38. In one example, without limitation, the medical end effector 38 is a medical grasper.

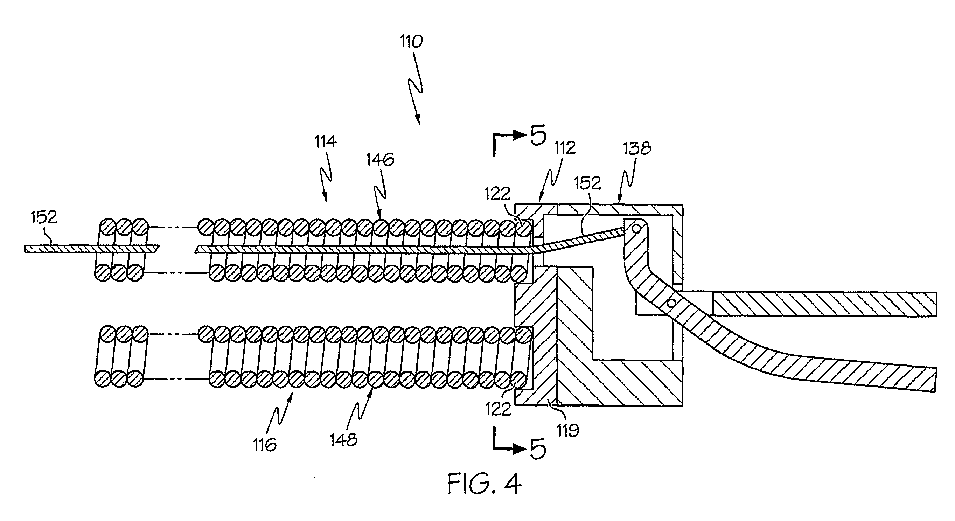

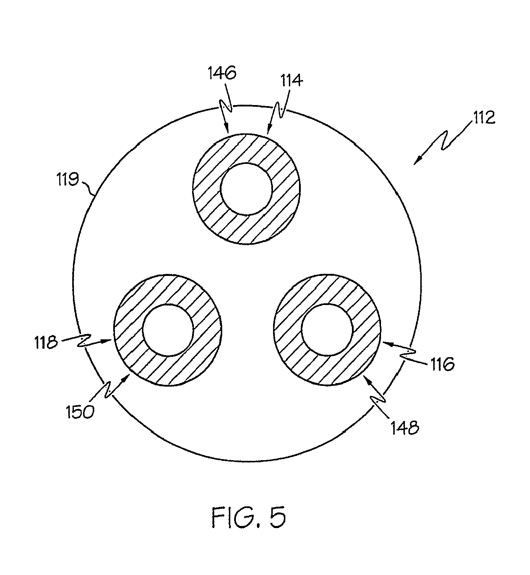

In a first different application, as seen in the first alternate embodiment of Figures 4-5, the medical instrument 110 includes a medical end effector 138 attached to the rigid portion 119 of the medical-end-effector-associated member 112. In one variation, the distal end portions 122 of the first, second and third elongate members 114, 116 and 118 are attached to the rigid portion 119 of the medical-end-effector-associated member 112. In one example, without limitation, the medical-end-effector-associated member 112 is a platform base, wherein articulation of the platform base articulates the attached medical end effector 138. In one illustration, the medical end effector 138 is a medical grasper.

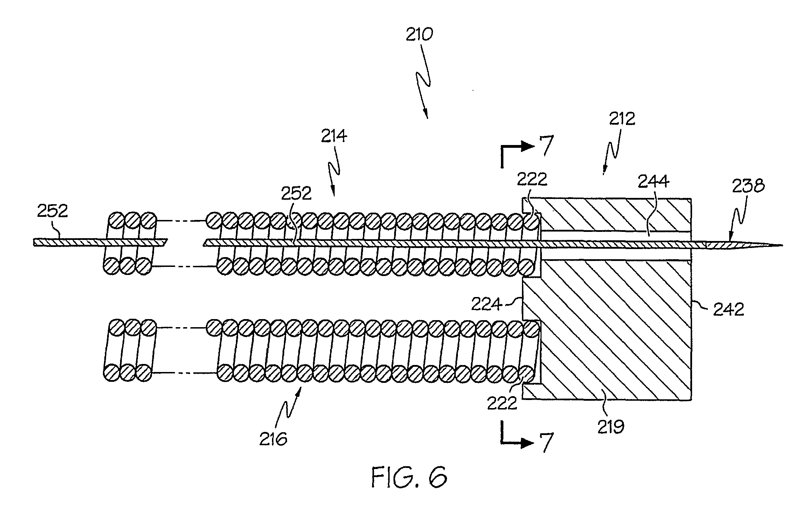

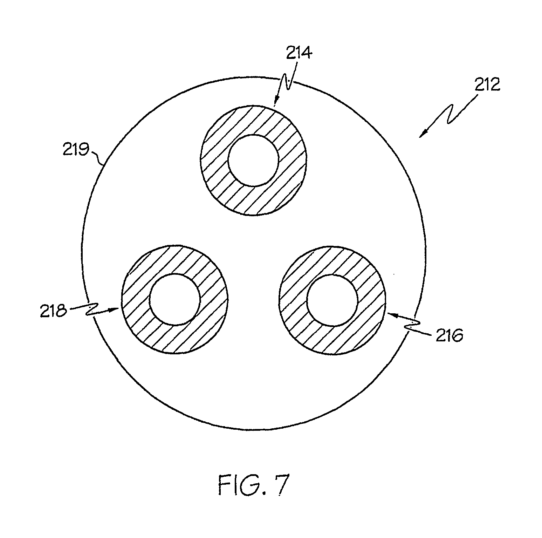

In a second different application, as seen in the second alternate embodiment of Figures 6-7, the rigid portion 219 of the medical-end-effector-associated member 212 has a proximal side 224, a distal side 242, and a passageway 244 extending from the proximal side 224 to the distal side 242, and the medical instrument 210 also includes a medical end effector 238 translatable through, distally extendable from, and proximally retractable into the passageway 244. In one variation, the distal end portions 222 of the first, second and third elongate members 214, 216 and 218 are attached to the rigid portion 219 of the medical-end-effector-associated member 212. In one example, without limitation, the medical-end-effector-associated member 212 is a platform guide, wherein articulation of the platform guide articulates the passageway 244 of the platform guide which reorients the medical end effector 238 when the medical end effector 238 is distally extended from the passageway 244. In one illustration, the medical end effector 238 is a medical needle-knife, such a radio-frequency-energized medical needle-knife.

In one extension of the first expression of the embodiment of Figures 1-3, the medical instrument 10 also includes a handpiece, not shown. In one example, the handpiece includes a joystick-type handle operatively connected to each of the proximal end portions 20 of the first, second and third elongate members 14, 16 and 18, wherein moving the joystick handle provides relative lengthwise translation of the proximal end portions which articulates the rigid portion 19 of the medical-end-effector-associated member 12. In another example, not shown, the handpiece has one stationary finger ring for support and has first and second and third slidable finger rings connected to a corresponding one of the proximal end portions 20 for lengthwise translation thereof. Other examples of handpieces and robotic operation of the medical instrument 10 are left to those skilled in the art. In a different employment, a user manually relatively translates the proximal end portions 20 of the first, second and third elongate members 14, 16 and 18 to articulate the medical-end-effector-associated member 12.

A second expression of the embodiment of Figures 1-3 is for a medical instrument 10 including a medical-end-effector-associated member 12 and resiliently flexible first, second and third coilpipes 46, 48 and 50. It is noted that a coilpipe is an example of an elongated member. The medical-end-effector-associated member 12 has a rigid portion 19 disposable within a patient. The first, second and third coilpipes 46, 48 and 50 each have a proximal end portion 20 and a distal end portion 22. The distal end portions 22 of the first, second and third coilpipes 46, 48 and 50 are attached to the rigid portion 19 of the medical-end-effector-associated member 12. Relative lengthwise translation of the proximal end portions 20 of the first, second and third coilpipes 46, 48 and 50 articulates the rigid portion 19 of the medical-end-effector-associated member 12.

In one deployment of the second expression of the embodiment of Figures 1-3, adjacent coil turns of a coilpipe contact each other. In a different deployment, adjacent coil turns of a coilpipe do not contact each other. Other deployments are left to the artisan. It is noted that the enablements, implementations, applications, etc. of the first expression of the embodiment of Figures 1-3 and the different applications thereof are equally applicable to the second expression of the embodiment of Figures 1-3, wherein it is noted that a coilpipe is an example of an elongated member.

A third expression of the embodiment of Figures 1-3 is for a medical instrument 10 including a medical-end-effector-associated member 12, resiliently flexible first, second and third coilpipes 46, 48 and 50, and a medical-end-effector activation cable 52. The medical-end-effector-associated member 12 has a rigid portion 19 disposable within a patient. The first, second and third coilpipes 46, 48 and 50 each have a proximal end portion 20 and a distal end portion 22. The distal end portions 22 of the first, second and third coilpipes 46, 48 and 50 are attached to the rigid portion 19 of the medical-end-effector-associated member 12. Relative lengthwise translation of the proximal end portions 20 of the first, second and third coilpipes 46, 48 and 50 articulates the rigid portion 19 of the medical-end-effector-associated member 12. The activation cable 52 is disposable in one of the first, second and third coilpipes 46, 48 and 50.

In one deployment of the third expression of the embodiment of Figures 1-3, adjacent coil turns of a coilpipe contact each other. In a different deployment, adjacent coil turns of a coilpipe do not contact each other. Other deployments are left to the artisan. It is noted that the enablements, implementations, applications, etc. of the first expression of the embodiment of Figures 1-3 and the different applications thereof are equally applicable to the third expression of the embodiment of Figures 1-3, wherein it is noted that a coilpipe is an example of an elongated member.

In one arrangement wherein the medical-end-effector-associated member 12 is a medical end effector 38, the activation cable 52 is disposed in one of the first, second and third coilpipes 46, 48 and 50 and is operatively connected to the medical end effector 38. In one arrangement wherein the medical instrument 110 also includes a medical end effector 138 attached to the medical-end-effector-associated member 112, the activation cable 152 is disposed in one of the first, second and third coilpipes 146, 148 and 150 and is operatively connected to the medical end effector 138. In one arrangement wherein the medical-end-effector-associated member 212 has a passageway 244, the medical instrument 210 also includes a medical end effector 238 attached to the activation cable 252, wherein distal lengthwise translation of the activation cable 252 distally extends the medical end effector 238 from the passageway 244 and proximal lengthwise translation of the activation cable 252 proximally retracts the medical end effector 238 into the passageway 244. In one choice of materials, the activation cable 52, 152 and 252 consists essentially of nitinol.

Several benefits and advantages are obtained from one or more of the expressions of an embodiment of the invention. In a first application, the medical-end-effector-associated member is inserted into a working channel of a flexible insertion tube of an endoscope, wherein the medical-end-effector-associated member can be articulated with respect to the insertion tube of the endoscope allowing independent alignment of the wide angle video camera of the endoscope and the medical-end-effector-associated member. In a second application, the medical-end-effector-associated member is adapted to be coupled to, and slid along, an exterior rail of a flexible insertion tube of an endoscope allowing independent alignment of the wide angle video camera of the endoscope and the medical-end-effector-associated member. In one example, the medical-end-effector-associated member is a medical end effector such as, without limitation, a medical grasper. In another example, the medical-end-effector-associated member has a passageway, and the medical instrument also includes a medical end effector, such as, without limitation, a medical needle-knife which is translatable through, distally extendable from, and proximally retractable into the passageway.

While the present invention has been illustrated by a description of several expressions of embodiments, it is not the intention of the applicants to restrict or limit the spirit and scope of the appended claims to such detail. Numerous other variations, changes, and substitutions will occur to those skilled in the art without departing from the scope of the invention. For instance, the medical instrument of the invention has application in robotic assisted surgery taking into account the obvious modifications of such systems, components and methods to be compatible with such a robotic system. It will be understood that the foregoing description is provided by way of example, and that other modifications may occur to those skilled in the art without departing from the scope and spirit of the appended Claims.

## Claims

1. A medical instrument comprising:
a) a medical-end-effector-associated member having a rigid portion disposable within a patient; and
b) resiliently flexible first, second and third elongate members each having a proximal end portion and a distal end portion, wherein the distal end portions of the first, second and third elongate members are attached to the rigid portion of the medical-end-effector-associated member, and wherein relative lengthwise translation of the proximal end portions of the first, second and third elongate members articulates the rigid portion of the medical-end-effector-associated member.

2. The medical instrument of claim 1, wherein the rigid portion of the medical-end-effector-associated member has a proximal side, and wherein the distal end portions of the first, second and third elongate members have centerlines which intersect the proximal side at points which define vertices of a substantially equilateral triangle.

3. The medical instrument of claim 1, wherein the medical-end-effector-associated member and the distal end portions of the first, second and third elongate members are disposable within a working channel of an endoscope insertion tube.

4. The medical instrument of claim 1, wherein the first, second and third elongate members consist essentially of nitinol.

5. The medical instrument of claim 1, wherein the medical-end-effector-associated member is a medical end effector.

6. The medical instrument of claim 5, also including a medical end effector attached to the rigid portion of the medical-end-effector-associated member.

7. The medical instrument of claim 1, wherein the rigid portion of the medical-end-effector-associated member has a proximal side, a distal side, and a passageway extending from the proximal side to the distal side, and also including a medical end effector translatable through, distally extendable from, and proximally retractable into the passageway.

8. A medical instrument comprising:
a) a medical-end-effector-associated member having a rigid portion disposable within a patient; and
b) resiliently flexible first, second and third coilpipes each having a proximal end portion and a distal end portion, wherein the distal end portions of the first, second and third coilpipes are attached to the rigid portion of the medical-end-effector-associated member, and wherein relative lengthwise translation of the proximal end portions of the first, second and third coilpipes articulates the rigid portion of the medical-end-effector-associated member.

9. The medical instrument of claim 8, wherein the rigid portion of the medical-end-effector-associated member has a proximal side, and wherein the distal end portions of the first, second and third coilpipes have coil centerlines which intersect the proximal side at points which define vertices of a substantially equilateral triangle.

10. The medical instrument of claim 8, wherein the medical-end-effector-associated member and the distal end portions of the first, second and third coilpipes are disposable within a working channel of an endoscope insertion tube.

11. A medical instrument comprising:
a) a medical-end-effector-associated member having a rigid portion disposable within a patient;
b) resiliently flexible first, second and third coilpipes each having a proximal end portion and a distal end portion, wherein the distal end portions of the first, second and third coilpipes are attached to the rigid portion of the medical-end-effector-associated member, and wherein relative lengthwise translation of the proximal end portions of the first, second and third coilpipes articulates the rigid portion of the medical-end-effector-associated member; and
c) a medical-end-effector activation cable disposable in one of the first, second and third coilpipes.
